# EUROPEAN PATENT APPLICATION

(11) **EP 4 545 974 A1**
(43) Date of publication of application: **30.04.2025**
(21) Application number: 23850247.0
(22) Date of filing: 19.06.2023
(51) Int. Cl.: G01N 35/00, G01N 11/00, G01N 33/49, G01N 35/10

(54) **AUTOMATIC BLOOD VISCOSITY MEASUREMENT DEVICE**

(30) Priority: 01.08.2022 KR 20220095507; 13.09.2022 KR 20220114900
(71) Applicant: INDUSTRIAL COOPERATION FOUNDATION JEONBUK NATIONAL UNIVERSITY, Jeonju-si, Jeollabuk-do 54896 (KR)
(72) Inventor: LEE, Dong Hwan, Jeonju-si Jeollabuk-do 54900 (KR)
(74) Representative: Weickmann & Weickmann PartmbB
(86) International application number: PCT/KR2023/008430
(87) International publication number: WO 2024/029737

(57) **Abstract**

An automatic blood viscosity measurement device includes a housing having an open door; a blood sample mounting unit on which multiple blood collection tubes are mounted; a blood sample preprocessing unit that grips each of the multiple blood collection tubes, scans a gripped blood collection tube, and separates a blood collection tube cover mounted on the gripped blood collection tube; a blood sample transfer unit that moves the blood collection tube gripped by the blood sample preprocessing unit; and a blood viscosity measurement unit on which a blood viscosity measurement kit is mounted and which includes one or more channels for measuring viscosity of a blood sample injected into the blood viscosity measurement kit, and thus, there is an advantage of being able to measure viscosity of one or more blood samples simultaneously, resulting in reduced working time and improved ease of use.

## Description

### Technical Field

The present invention relates to an automatic blood viscosity measurement device, and particularly to an automatic blood viscosity measurement device that may measure the viscosity of one or more blood samples simultaneously by providing multiple modules for transferring and measuring the blood samples, thereby reducing the work time and improving ease of use.

### Background Art

Blood viscosity is a physical property that represents the flow resistance due to the flow of blood in blood vessels and may be specifically divided into whole blood viscosity and plasma viscosity. An abnormal increase in blood viscosity causes an increase in shear stress and flow resistance acting on inner walls of blood vessels, which significantly increases the risk of developing acute cardiovascular disease and microvascular disease.

In addition, the plasma viscosity is not only used to diagnose inflammatory conditions in the body, but also is one of the main causes of increased whole blood viscosity.

The whole blood viscosity shows flow characteristics in which viscosity continuously changes according to the systole and diastole of the heart, and the reason is because the viscosity decreases when the blood flows at a high speed (when a shear rate is high) due to the mutual complex influence of red blood cells and plasma proteins in the whole blood, and conversely, the viscosity increases when the blood flows at a low speed (when the shear rate is low). A fluid that shows the flow characteristics is called a non-Newtonian fluid, and in order to properly understand the non-Newtonian flow characteristics of blood, it is necessary to accurately measure the whole blood viscosity for the entire shear rate (for example, 1 to 1,000 s^-1).

Recent blood viscosity measurement devices allow the blood obtained from the body to pass through a flow restrictor tube, and measure the flow characteristics of blood within the flow restrictor tube to measure blood viscosity and blood cell aggregation rate.

Korean Utility Model No. 20-0331884 (Apparatus to measure simultaneously both blood viscosity and cell aggregation) which is a conventional technology is disclosed.

However, since a device operator has to manually inject blood through a syringe to measure blood viscosity, it is difficult to supply blood at a constant pressure and flow rate, and thus, there is a problem that it is difficult to measure blood viscosity under the same condition.

In addition, since it is done manually, there is a problem that the work time is lengthened, and since it is done manually, a blood-borne infection problem also occurs frequently.

In order to solve the problem, an automated blood viscosity measurement device is being developed, but there is a problem that the work time is lengthened because when the viscosity measurement for one blood sample is completed, the viscosity measurement for the next blood sample is performed.

In addition, when measuring a large number of blood samples, in a case where the blood sample is a late-order blood sample, the viscosity measurement is performed in a state where red blood cells, or the like settle over time, resulting in a problem of reduced accuracy of the viscosity measurement.

In addition, since the conventional blood viscosity measurement device has only one gripper and measurement module, when one gripper module breaks down, the entire measurement device stops operating, and accordingly, in order to solve this problem, it is necessary to develop technology for an automatic blood viscosity measurement device with excellent maintenance power by having multiple grippers.

### Technical Problem

The present invention is designed to solve the above-described problem, and an objective of the present invention is to provide an automatic blood viscosity measurement device that is configured to uniformly perform the entire process from blood sample injection to mixing, suction, dispensing, and disposal through an automated robot system, thereby improving measurement speed and measurement accuracy and improving ease of use by including multiple modules for transferring and measuring blood samples.

### Technical Solution

An automatic blood viscosity measurement device according to an embodiment of the present invention includes a housing including an open door; a blood sample mounting unit on which multiple blood collection tubes are mounted; a blood sample preprocessing unit that grips each of the multiple blood collection tubes, scans a gripped blood collection tube, and separates a blood collection tube cover mounted on the gripped blood collection tube; a blood sample transfer unit that moves the blood collection tube gripped by the blood sample preprocessing unit; a blood viscosity measurement unit on which a blood viscosity measurement kit is mounted and which includes one or more channels for measuring viscosity of a blood sample injected into the blood viscosity measurement kit; a kit mounting unit provided inside the housing for mounting the blood viscosity measurement kit on the blood viscosity measurement unit; and a blood sample postprocessing unit that sucks a blood sample of a blood collection tube transferred by the blood sample transfer unit and injects the blood sample into a mounted blood viscosity measurement kit.

Here, the blood sample mounting unit may include a blood collection tube insertion plate on which the blood collection tube is vertically mounted, a first sliding unit that slides the blood collection tube insertion plate to a front of the housing to expose the blood collection tube insertion plate to an outside, and a first detection unit provided between the blood collection tube insertion plate and the first sliding unit to detect a safe-placement state of the blood collection tube insertion plate.

Also, the blood sample mounting unit further may include a motion motor provided on both sides of the blood collection tube insertion plate to prevent sedimentation of red blood cells of the blood sample in the blood collection tube by tilting the blood collection tube insertion plate at a set angle to left and right and repeatedly moving the blood collection tube insertion plate at a set cycle.

Also, the blood sample preprocessing unit may include a preprocessing gripper provided in multiple units to grip the blood collection tube and the blood collection tube cover to rotate the blood collection tube and the blood collection tube cover, a first transfer module for adjusting a horizontal position of the preprocessing gripper, and a first lifting/lowering module causing the preprocessing gripper to be associated with the first transfer module and adjusting a height of the preprocessing gripper.

Here, the blood sample preprocessing unit may further include a scanning module provided in the first transfer module to scan whether the blood collection tube is mounted on the blood collection tube insertion plate.

Also, the blood sample transfer unit may include a holding gripper which is provided in multiple units and in which the blood collection tube being gripped and transferred by the preprocessing gripper is safely placed and which grips a lower portion of the blood collection tube, a holding gripper transfer module that transfers the holding gripper, and a guide plate that guides a reciprocating movement of the holding gripper transferred by the holding gripper transfer module.

Also, the blood sample preprocessing unit may further include a blood collection tube detection unit that detects one or more blood collection tubes gripped by the holding gripper, and the automatic blood viscosity measurement device may automatically measure viscosity of one or more blood samples of the one or more blood collection tubes according to detection information on the one or more blood collection tube.

Also, the blood viscosity measurement unit may include a measurement unit body having a vibration-proof pad installed at an upper portion of the measurement unit; a kit mounting housing provided at an upper end of the measurement unit body and having a kit insertion groove in which the blood viscosity measurement kit is mounted; a mounting detection sensor provided inside the kit insertion groove and detecting a mounting state of the blood viscosity measurement kit; a heat transfer member provided inside the kit mounting housing to apply heat to the blood viscosity measurement kit; and a kit fixing means provided inside the kit mounting housing to place at a normal position and fix the blood viscosity measurement kit, and the kit fixing means may include a first push member provided on an inner surface of the kit insertion groove of the kit mounting housing in a height direction of the inner surface to push the blood viscosity measurement kit to another side; and a second push member provided on the inner surface of the kit insertion groove of the kit mounting housing in a length direction of the inner surface to push the blood viscosity measurement kit to another side.

Also, the kit mounting unit may include a kit insertion tray on which the blood viscosity measurement kit is vertically mounted; a second sliding unit that slides the kit insertion tray to a front of the housing to expose the kit insertion tray to the outside; and a second detection unit provided between the kit insertion tray and the second sliding unit to detect a safe-placement state of the kit insertion tray.

Here, the kit mounting unit further may include a fastening means provided between the kit insertion tray and the second sliding unit to fix the kit insertion tray to an upper portion of the kit insertion tray.

Also, the kit mounting unit may be provided in multiple units and include a kit gripper that grips the blood viscosity measurement kit; a second transfer module that adjusts a horizontal position of the kit gripper; and a second lifting/lowering module causing the kit gripper to be associated with the second transfer module and adjusting a height of the kit gripper.

Also, the blood sample postprocessing unit may be provided in multiple units and include an injection device that mixes and sucks the blood sample of the blood collection tube transferred by the blood sample transfer unit in association with the second transfer module and injects the blood sample into the mounted blood viscosity measurement kit.

Also, the blood sample postprocessing unit may further include a pipette tip insertion tray on which a pipette tip that is detachable from an end portion of the injection device is mounted, and a third sliding unit that slides the pipette tip insertion tray to a front of the housing to expose the pipette tip insertion tray to an outside.

Also, the housing may include a first waste unit provided between the blood sample mounting unit and the blood viscosity measurement unit to accommodate a used pipette tip, and a second waste unit provided between the blood viscosity measurement unit and the kit mounting unit to accommodate a blood viscosity measurement kit for which measurement is completed, and the first waste unit and the second waste unit may be exposed to an outside of the housing by sliding forward the housing.

Also, the automatic blood viscosity measurement device may further include a control unit for controlling operations of the blood sample preprocessing unit, the blood sample transfer unit, the blood viscosity measurement unit, the kit mounting unit, and the blood sample postprocessing unit; an alarm unit for detecting and notifying an error of the automatic blood viscosity measurement device; an emergency stop switch unit for urgently stopping the automatic blood viscosity measurement device; and a monitor unit for monitoring a viscosity measurement result of the blood viscosity measurement unit.

### Advantageous Effects

An automatic blood viscosity measurement device according to an embodiment of the present invention uses a configuration of a dual system, and thus, there is an advantage of shortening a blood viscosity test time and enabling continuous testing even when a gripper module or an injection device fails.

In addition, in order to prevent sedimentation of red blood cells during the test, a blood sample may be mixed by using pipetting to evenly disperse red blood cells and so on before sucking blood, along with a movement of a blood collection tube insertion plate, and thus, more accurate blood viscosity measurement may be performed.

In addition, the automatic blood viscosity measurement device according to an embodiment of the present invention enables safe and quick replacement of a blood collection tube, a blood viscosity measurement kit, and a pipette tip.

In addition, an automatic blood viscosity measurement device according to an embodiment of the present invention is not limited to the effects described above, and it is preferable to interpret that the automatic blood viscosity measurement device has an advantage that may be obviously derived through a combination of devices and configurations of the present invention.

### Description of Drawings

FIG. 1 is a perspective view of an automatic blood viscosity measurement device according to an embodiment of the present invention.
FIG. 2 is an example view illustrating an open door and a front portion of a housing with removed some of the housing.
FIG. 3 is a perspective view illustrating the entire configuration of a blood sample mounting unit, a blood sample preprocessing unit, a blood sample transfer unit, a blood viscosity measurement unit, a kit mounting unit, and a blood sample postprocessing unit which are built in a housing.
FIG. 4 is a front view of FIG. 3.
FIG. 5 is a rear view of FIG. 3.
FIG. 6 is a perspective view of a blood sample mounting unit and a blood sample transfer unit according to an embodiment of the present invention.
FIG. 7 is an example view of a blood collection tube insertion plate equipped with a motion motor.
FIG. 8 is an example view of a shape in which a blood collection tube insertion plate and a guide plate are removed from FIG. 6.
FIG. 9 is an example view illustrating a configuration of a first sliding unit.
FIG. 10 is a perspective view of a blood sample preprocessing unit, a blood viscosity measurement unit, a kit mounting unit, and a blood sample postprocessing unit.
FIG. 11 is a perspective view of a blood viscosity measurement unit.
FIG. 12 is an example view illustrating a shape of a measurement unit body.
FIG. 13 is an example view of a kit mounting housing.
FIG. 14 is an example view of a kit mounting housing provided with a heat transfer member and a temperature detection sensor.
FIG. 15 is an example view of a kit mounting housing provided with a kit fixing means.
FIG. 16 is an example view illustrating a configuration of a second sliding unit.
FIG. 17 is a perspective view of a kit mounting unit provided with a second detection unit.
FIG. 18 is an example view of a kit gripper and an injection device mounted on a second transfer module.
FIG. 19 is an example view illustrating a configuration of a third sliding unit.
FIG. 20 is an example view illustrating a third detection unit.

### Detailed Description of Invention

An automatic blood viscosity measurement device according to an embodiment of the present invention includes a housing including an open door; a blood sample mounting unit on which multiple blood collection tubes are mounted; a blood sample preprocessing unit that grips each of the multiple blood collection tubes, scans a gripped blood collection tube, and separates a blood collection tube cover mounted on the gripped blood collection tube; a blood sample transfer unit that moves the blood collection tube gripped by the blood sample preprocessing unit; a blood viscosity measurement unit on which a blood viscosity measurement kit is mounted and which includes one or more channels for measuring viscosity of a blood sample injected into the blood viscosity measurement kit; a kit mounting unit provided inside the housing for mounting the blood viscosity measurement kit on the blood viscosity measurement unit; and a blood sample postprocessing unit that sucks a blood sample of a blood collection tube transferred by the blood sample transfer unit and injects the blood sample into a mounted blood viscosity measurement kit.

### Mode for Invention

Hereinafter, descriptions of the present invention made with reference to the drawings are not limited to specific embodiments, and various changes may be made and various embodiments may be derived. Also, the content described below should be understood to include all conversions, equivalents, and substitutes included in the idea and technical scope of the present invention.

In the following description, terms "first", "second", and so on are used to describe various components, and their meaning is not limited, and is used only for the purpose of distinguishing one component from other components.

Like reference numerals used throughout the present invention refer to like elements.

As used herein, singular expressions include plural expressions, unless the context clearly dictates otherwise. Also, terms such as "include", "provide", or "have" used below should be construed as intended to designate the presence of features, numbers, steps, operations, components, portions, or a combination thereof described in the specification and should be understood as not precluding the presence or addition of one or more other features, numbers, steps, operations, components, parts or combinations thereof.

Unless otherwise defined, all terms used herein, including technical or scientific terms, have the same meaning as generally understood by those skilled in the technical field to which the present invention pertains. Terms such as those defined in commonly used dictionaries should be interpreted as having meanings consistent with the meanings they have in the context of the related technology, and should not be interpreted as having ideal or excessively formal meanings, unless explicitly defined in the present application.

Also, when describing with reference to the accompanying drawings, identical components are assigned the same reference numerals regardless of the reference numerals, and overlapping descriptions thereof are omitted. When it is determined that a detailed description of related known technologies may unnecessarily obscure the gist of the present invention in describing the present invention, the detailed descriptions are omitted.

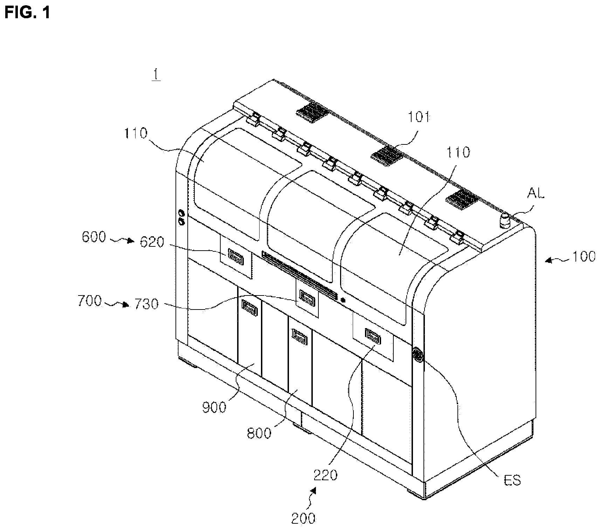

Hereinafter, an automatic blood viscosity measurement device according to an embodiment of the present invention will be described in detail with reference to FIG. 1 to FIG. 20, and specific embodiments will be described together.

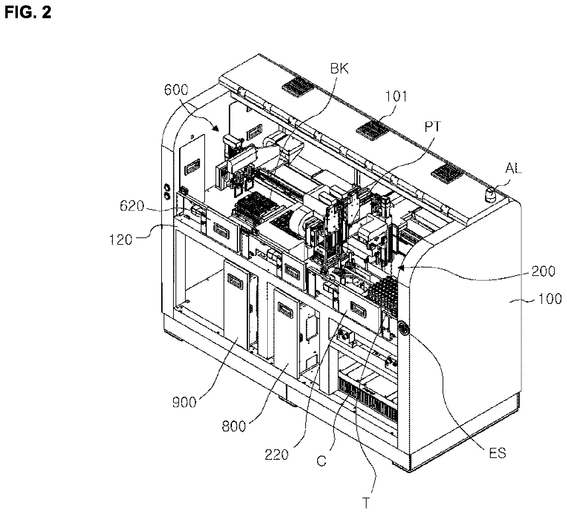

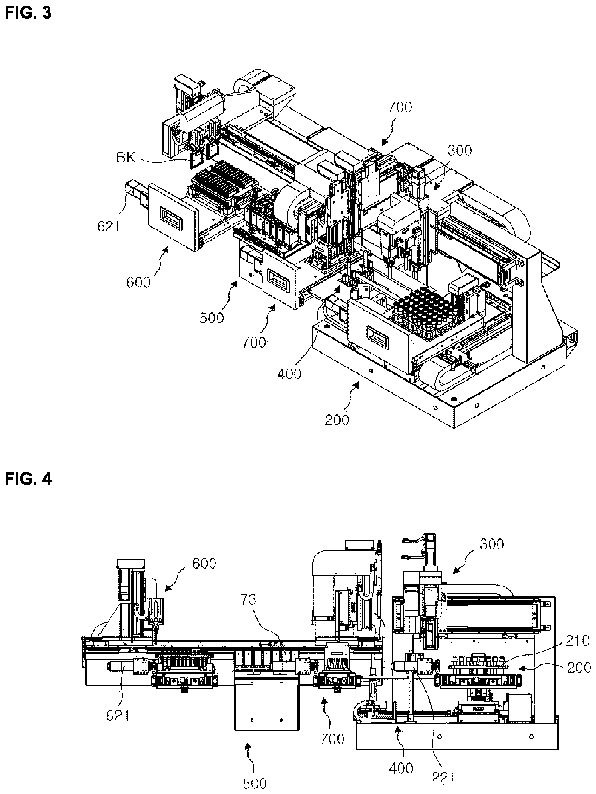

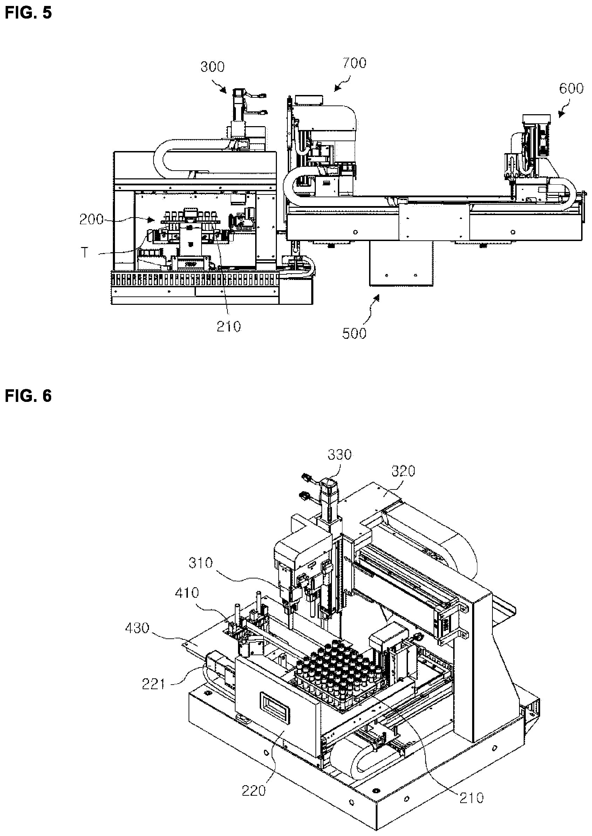

First, FIG. 1 is a perspective view of an automatic blood viscosity measurement device according to an embodiment of the present invention, FIG. 2 is an example view illustrating an open door and a front portion of a housing with removed some of the housing, FIG. 3 is a perspective view illustrating the entire configuration of a blood sample mounting unit, a blood sample preprocessing unit, a blood sample transfer unit, a blood viscosity measurement unit, a kit mounting unit, and a blood sample postprocessing unit which are built in a housing, FIG. 4 is a front view of FIG. 3, and FIG. 5 is a rear view of FIG. 3.

Referring to FIG. 1 to FIG. 5, an automatic blood viscosity measurement device 1 according to an embodiment of the present invention may include a housing 100, a blood sample mounting unit 200, a blood sample preprocessing unit 300, a blood sample transfer unit 400, a blood viscosity measurement unit 500, a kit mounting unit 600, and a blood sample postprocessing unit 700.

First, the housing 100 refers to a housing forming the exterior of the automatic blood viscosity measurement device 1, and may have a space for respective mechanical devices described below to be built in.

In addition, the housing 100 may be provided with a cooling unit 101 for discharging the heat generated from respective mechanical devices to the outside, and more specifically, the cooling unit 101 may be formed by a combination of an air inlet that is provided at an upper portion of the housing 100 to suck air from the outside into the inside of the housing 100 and an air outlet provided at the rear of the housing 100 to discharge the sucked air to the outside.

In addition, an open door 110 may be provided at the front of the housing 100, and at this time, the open door 110 may be provided at the front and rear radii of the blood sample mounting unit 200, the kit mounting unit 600, and the blood sample postprocessing unit 700, and may be formed of a transparent material such as acrylic such that an operation of a mechanical device may be identified from the outside, and further, the open door 110 may be provided with a locking device to resolve a problem that a device is unexpectedly opened during operation.

In addition, the housing 100, blood sample mounting unit 200, the blood sample preprocessing unit 300, the blood sample transfer unit 400, the blood viscosity measurement unit 500, the kit mounting unit 600, and the blood sample postprocessing unit 700 may be mounted inside the housing 100, and a frame 120 for forming a supporting force may be provided.

At this time, the frame 120 may be a structure that easily supports loads of respective mechanical devices and also has a vibration attenuation structure for an external impact or the vibration transmitted from the ground.

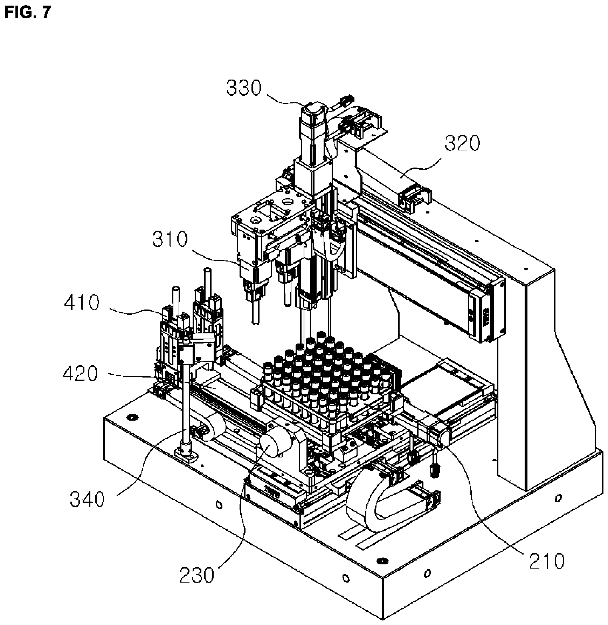

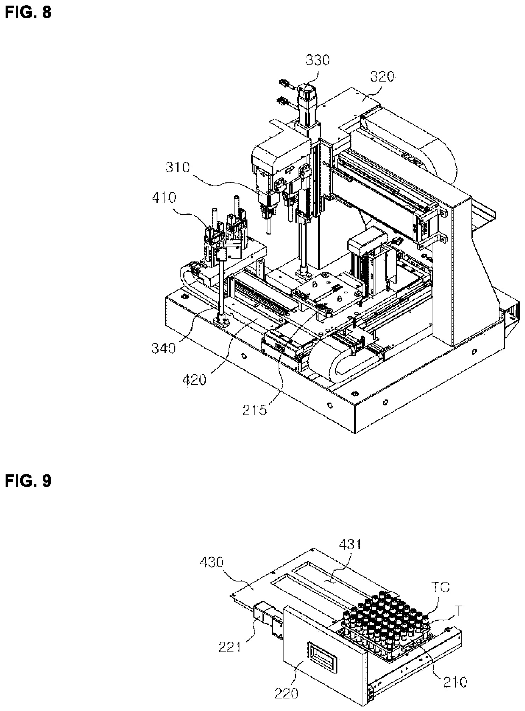

Next, FIG. 6 is a perspective view of a blood sample mounting unit and a blood sample transfer unit according to an embodiment of the present invention, FIG. 7 is an example view of a blood collection tube insertion plate equipped with a motion motor, FIG. 8 is an example view of a shape in which the blood collection tube insertion plate and a guide plate are removed from FIG. 6, and FIG. 9 is an example view illustrating a configuration of a first sliding unit.

Referring to FIG. 6 to FIG. 9, the blood sample mounting unit 200 according to an embodiment of the present invention may be mounted with a blood collection tube T containing a blood sample to be measured, and at this time, the blood collection tube T is a container having a length as illustrated in FIG. 9 and may be provided with a blood collection tube cover TC on an upper portion to seal the blood collection tube T from the outside.

Specifically, the blood sample mounting unit 200 according to an embodiment of the present invention may include a blood collection tube insertion plate 210, a first sliding unit 220, and a first detection unit 215.

First, the blood collection tube T having a length may be mounted vertically on the blood collection tube insertion plate 210, but this is only a layout structure for a preprocessing gripper 310 described below to easily grip the blood collection tube T, and the blood collection tube T may also be mounted in a horizontally lying shape according to a user's request.

In addition, the first sliding unit 220 may expose the blood collection tube insertion plate 210 to the outside by sliding the blood collection tube insertion plate 210 to the front of the housing 100, and a general shape thereof may be provided in a shape similar to a sliding drawer of a desk.

In addition, a first locking device 221 may be provided on a side of the first sliding unit 220 to prevent the first sliding unit 220 from sliding unexpectedly during an operation of a device.

In addition, the first detection unit 215 is provided between the blood collection tube insertion plate 210 and the first sliding unit 220, and may detect a safe-placement state of the blood collection tube insertion plate 210.

At this time, the blood sample mounting unit 200 may further include a motion motor 230 provided on both sides of the blood collection tube insertion plate 210 to move the blood collection tube insertion plate 210 at a set cycle, and at this time, the motion motor 230 may be provided in a form built in the first sliding unit 220 within a range that does not interfere with a sliding movement of the first sliding unit 220.

Therethrough, the blood sample mounting unit 200 according to the embodiment of the present invention has an advantage of preventing sedimentation of red blood cells in a blood sample through a process of mixing by shaking the blood collection tube insertion plate 210 to the left and right.

In addition, the blood sample preprocessing unit 300 may grip multiple blood collection tubes T, scan a barcode attached to the blood collection tube T that is gripped in association with the blood collection tube detection unit 340, and separate the blood collection tube cover TC mounted on the top of the blood collection tube T.

In order to perform the operation described above, the blood sample preprocessing unit 300 according to the embodiment of the present invention may include the preprocessing gripper 310, a first transfer module 320, and a first lifting/lowering module 330.

First, the preprocessing gripper 310 may be provided in multiple units and may grip and rotate the blood collection tube T and the blood collection tube cover TC, thereby separating the blood collection tube cover TC from the blood collection tube T.

In addition, the first transfer module 320 may control a horizontal position of the preprocessing gripper 310, and may be formed to have a length in a measurement direction at the rear of the preprocessing gripper 310 as illustrated in FIG. 6.

At this time, the first transfer module 320 may further include a scanning module (not illustrated) that scans whether a blood collection tube is mounted on the blood collection tube insertion plate 210.

Specifically, the scanning module (not illustrated) ban be equipped with a recognition device including a laser or photo sensor in the first transfer module 320 that controls a horizontal position of the preprocessing gripper 310 and may automatically scan whether the blood collection tube T mounted in each hole of the blood collection tube insertion plate 210 is mounted.

In addition, the first lifting/lowering module 330 may control a height of the preprocessing gripper 310 by causing the preprocessing gripper 310 to be associated with the first transfer module 320.

Meanwhile, a control motor (not illustrated) for adjusting a separation distance between the preprocessing gripper 310 and the first lifting/lowering module 330 may be provided between the preprocessing gripper 310 and the first lifting/lowering module 330, and therethrough, the preprocessing gripper 310 according to the embodiment of the present invention may perform horizontal and vertical movements as well as forward and backward movements.

In addition, the blood sample transfer unit 400 may move the blood collection tube T gripped by the blood sample preprocessing unit 300.

Specifically, the blood sample transfer unit 400 according to the embodiment of the present invention may include a holding gripper 410, a holding gripper transfer module 420, and a guide plate 430.

First, the holding gripper 410 may be provided in the same number as the preprocessing gripper 310, that is, in multiple units, and safely place the blood collection tube T gripped and transferred by the preprocessing gripper 310, and grip, that is, fix a lower portion of the blood collection tube T such that the blood collection tube cover TC may be easily separated.

In addition, the holding gripper transfer module 420 may transfer the holding gripper 410 and may be formed to have a length in a measurement direction at the bottom of the blood sample mounting unit 200, as illustrated in FIG. 8.

In addition, the guide plate 430 may guide a reciprocating motion of the holding gripper 410 transferred by the holding gripper transfer module 420, and to this end, a guide groove 431 may be formed on a surface of the guide plate 430 through which the holding gripper 410 may reciprocate.

In addition, the blood sample preprocessing unit 300 according to an embodiment of the present invention may include the blood collection tube detection unit 340 that detects one or more blood collection tubes T gripped by the holding gripper 410.

More specifically, the blood collection tube detection unit 340 may be provided on a horizontal line or a lifting and lowering path of a preprocessing gripper 310 gripping the blood collection tube T, and may be provided with a configuration of a barcode reader capable of recognizing barcode data attached to a circumference of the blood collection tube T, and may scan and detect the barcode data attached to a surface of the blood collection tube T as the gripped blood collection tube T rotates from the holding gripper 410.

Therethrough, the automatic blood viscosity measurement device 1 according to the embodiment of the present invention may automatically measure the viscosity of a blood sample of the blood collection tube T according to the detection information of the blood collection tube T.

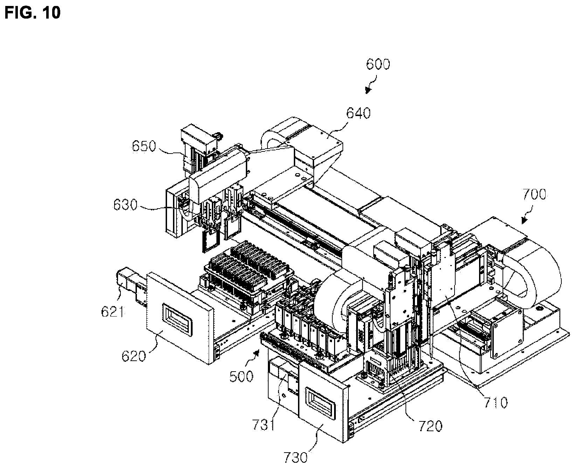

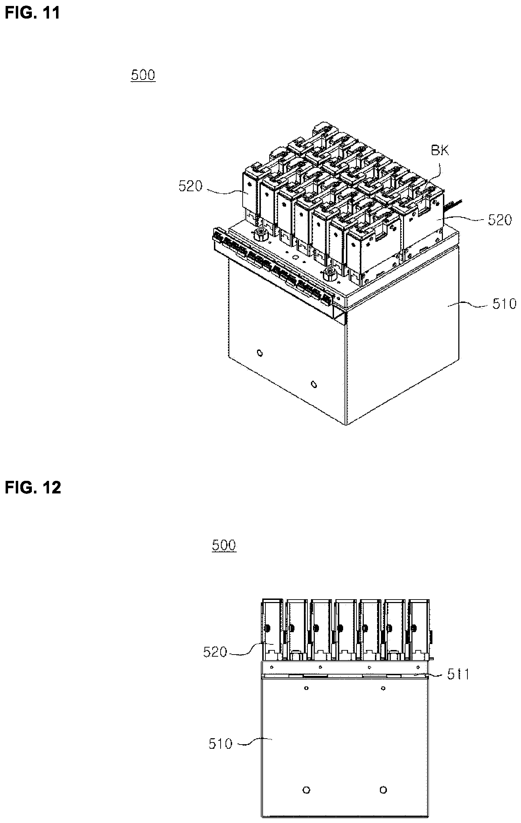

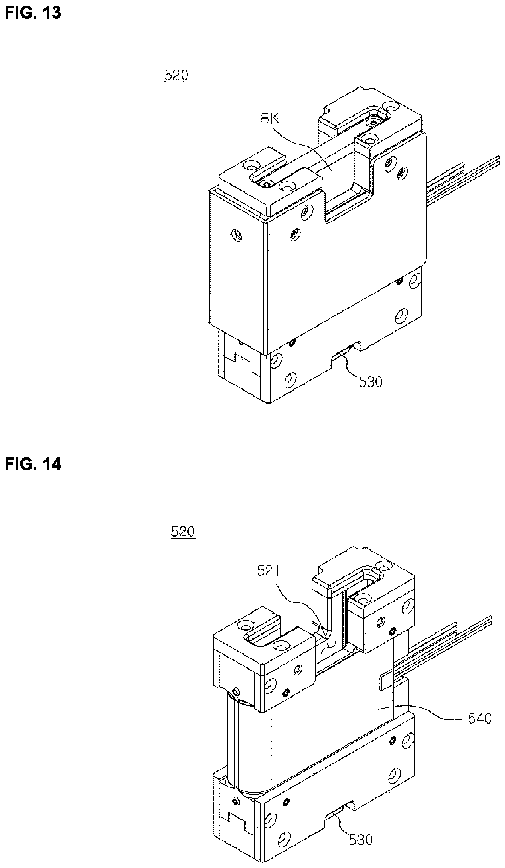

In addition, FIG. 10 is a perspective view of a blood sample preprocessing unit, a blood viscosity measurement unit, a kit mounting unit, and a blood sample postprocessing unit, FIG. 11 is a perspective view of the blood viscosity measurement unit, FIG. 12 is an example view illustrating a shape of a measurement unit body, FIG. 13 is an example view of a kit mounting housing, and FIG. 14 is an example view of a kit mounting housing provided with a heat transfer member and a temperature detection sensor

Referring to FIG. 10 and FIG. 11, the blood viscosity measurement unit 500 according to an embodiment of the present invention may include a blood viscosity measuring kit BK mounted thereon and may include one or more channels for measuring the viscosity of a blood sample injected into the blood viscosity measuring kit BK.

Specifically, the blood viscosity measurement unit 500 according to the embodiment of the present invention may include a measurement unit body 510, a kit mounting housing 520, a mounting detection sensor 530, a heat transfer member 540, and a kit fixing means 550.

First, the measurement unit body 510 may have a vibration-proof pad 511 installed on an upper portion thereof, and more specifically, the measurement unit body 510 may have two layers as illustrated in FIG. 12, and the vibration-proof pad 511 may be installed between the two layers to prevent vibration.

At this time, the vibration-proof pad 511 may be formed of a synthetic material such as rubber or silicone, preferably having two or more different elastic coefficients but is not limited thereto.

In addition, referring to FIG. 13 and FIG. 14, the kit mounting housing 520 is installed on an upper portion of the measurement unit body 510 and has a kit insertion groove 521 in which the blood viscosity measurement kit BK is mounted.

At this time, the kit mounting housing 520 is preferably provided with 14 channels, that is, 14 units, on an upper portion of the measurement unit body 510 but is not limited thereto, and the number of the kit mounting housings 520 may be expanded to 28 units or 56 units, depending on a size of the housing 100 or the specification of a power device of the present invention.

In addition, the mounting detection sensor 530 may be provided inside the kit insertion groove 521 and detect a mounting state of the blood viscosity measurement kit BK, and any electronic sensor that may detect a mounting state, such as a photo recognition sensor, a pressure point recognition sensor, or an ultrasonic recognition sensor, may be used as the mounting detection sensor 530.

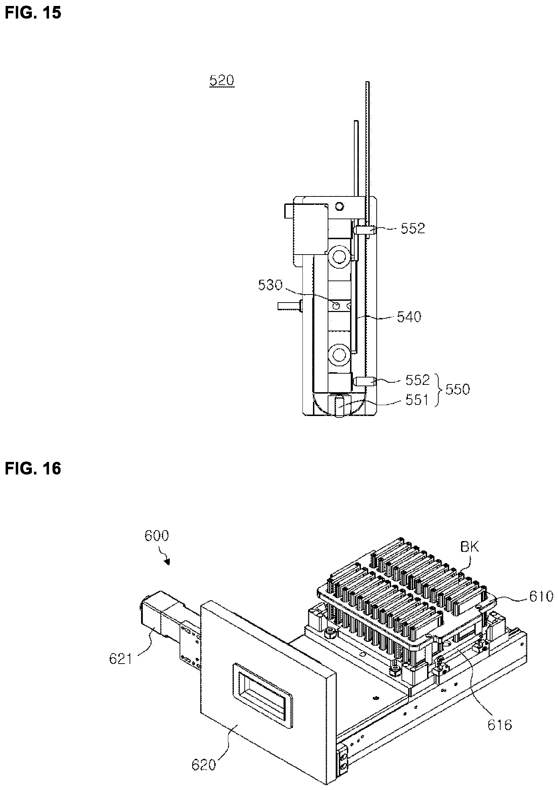

Meanwhile, the mounting detection sensor 530 may be provided at a lower center inside the kit insertion groove 521 as illustrated in FIG. 15, but this is only an exemplary form, and the mounting detection sensor 530 may also be formed on an inner side surface of the kit insertion groove 521.

In addition, the heat transfer member 540 may be provided inside the kit mounting housing 520 to apply heat to the blood viscosity measurement kit BK, and may provide overheating of the heat transfer member 540 by using a temperature sensor provided inside or outside the heat transfer member 540 and may also apply heat to the blood viscosity measurement kit BK in a user-defined temperature range.

Specifically, the heat transfer member 540 may be bent in a 'U' shape when viewed from a flat surface of the kit mounting housing 520 and may surround the blood viscosity measurement kit BK, or may be formed as a heat wire arranged in a zigzag repeating pattern, that is, in a waveform shape, around a circumference of the blood viscosity measurement kit BK.

Therethrough, the blood viscosity measurement unit 500 according to the embodiment of the present invention may effectively control the temperature of the mounted blood viscosity measuring kit BK, and thus, there is an advantage of being able to measure the blood viscosity more accurately.

In addition, the kit fixing means 550 may be provided inside the kit mounting housing 520 to place and fix the blood viscosity measuring kit BK on a blood height measurement sensor provided inside the kit insertion groove 521.

At this time, a (contact image sensor (CIS), which is an LED light sensor, may be used, as the blood height measurement sensor, but the present invention is not limited thereto, and any recognition sensor used to measure a blood height may be used.

More specifically, the kit fixing means 550 may be provided through a combination of a first push member 551 and a second push member 552.

First, the first push member 551 may be provided in a height direction of an inner surface of the kit insertion groove 521 of the kit mounting housing 520 and may push the blood viscosity measurement kit BK to the other side by the elasticity of a spring built therein.

In addition, the second push member 552 may be provided in a length direction of the inner surface of the kit insertion groove 521 of the kit mounting housing 520 and may push the blood viscosity measurement kit BK to the other side by the elasticity of a spring built therein.

In addition, the first push member 551 and the second push member 552 may each be provided in a single or multiple units inside the kit mounting housing 520 according to a user's needs, and it may be preferable that at least one of the two push members 551 and 552 is provided in multiple units.

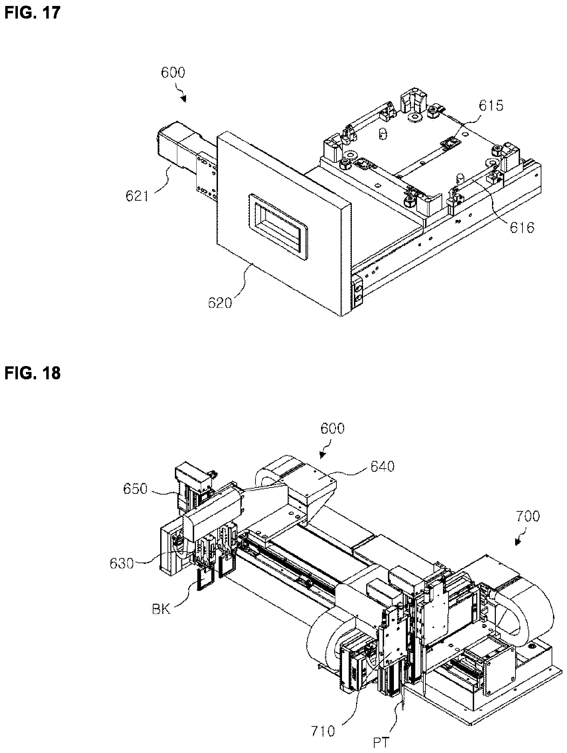

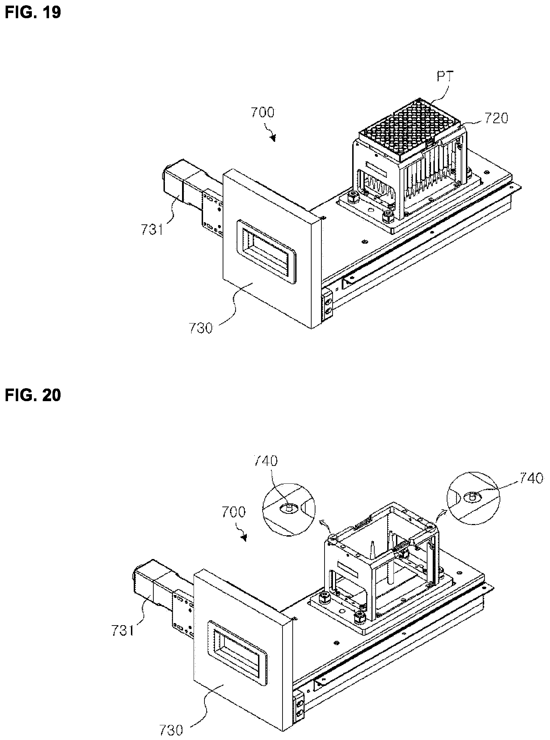

In addition, FIG. 16 is an example view illustrating a configuration of a second sliding unit, FIG. 17 is a perspective view of a kit mounting unit provided with a second detection unit, FIG. 18 is an example view of a kit gripper and an injection device mounted on a second transfer module, FIG. 19 is an example view illustrating a configuration of a third sliding unit, and FIG. 20 is an example view illustrating a third detection unit.

In addition, the kit mounting unit 600 may be provided inside the housing 100 and is equipped with the blood viscosity measurement kit BK such that the blood viscosity measurement kit BK may be mounted on the blood viscosity measurement unit 500, and at this time, the kit mounting unit 600 may also have configurations of the mounting sensors described above and may automatically scan whether the blood viscosity measurement kit BK is mounted, by using a recognition means such as a laser or a photo sensor.

More specifically, the kit mounting unit 600 may further include a kit insertion tray 610, a second sliding unit 620, a second detection unit 615, a kit gripper 630, a second transfer module 640, and a second elevating/lowering module 650.

First, a blood viscosity measurement kit BK may be vertically mounted on the kit insertion tray 610, and similarly to the description of the blood collection tube insertion plate 210 described above, the kit gripper 630 to be described below in detail may easily grip the blood viscosity measurement kit BK, and the blood viscosity measurement kit BK may be mounted in a horizontally lying form according to a user's request.

In addition, the second sliding unit 620 may expose the kit insertion tray 610 to the outside by sliding the kit insertion tray 610 to the front of the housing 100 and may be provided in the form of a sliding drawer similarly to the first sliding unit 220.

In addition, a second locking device 621 may be provided on a side of the second sliding unit 620 to prevent the second sliding unit 620 from sliding unexpectedly during an operation of a device.

In addition, the second detection unit 615 may be provided between the kit insertion tray 610 and the second sliding unit 620 to detect a safe-placement state of the kit insertion tray 610.

To this end, the second detection unit 615 may be provided with a configuration of a three-point mounting sensor as illustrated in FIG. 17, but this is only one type of methods for effectively detecting a horizontal state and mounting state of the kit insertion tray 610, and any electronic sensor that may effectively detect the horizontal and mounting states of the kit insertion tray 610 may be used.

In addition, a fastening means 616 may be provided between the kit insertion tray 610 and the second sliding unit 620 to stably fix the kit insertion tray 610 to an upper portion of the kit insertion tray 610, and more specifically, the fastening means 616 may be provided in a shape of a bar that rotates on the left and right sides of the kit insertion tray 610, as illustrated in FIGS. 16 and 17, and accordingly, it is possible to prevent the kit insertion tray 610 from shaking due to the second lifting/lowering module 650.

In addition, the kit gripper 630 may be provided in multiple units to be able to grip multiple blood viscosity measurement kits BK at once, and a pair of forceps included in a configuration thereof may be brought close to each other by a power device to grip the blood viscosity measurement kit BK.

In addition, the second transfer module 640 may control a horizontal position of the kit gripper 630 and may have a length inside the housing 100 in a measurement direction, like the first transfer module 320.

In addition, the second elevating/lowering module 650 may control a height of the kit gripper 630 by causing the kit gripper 630 to be associated with the second transfer module 640.

At this time, a control motor (not illustrated) for adjusting a separation distance between the second transfer module 640 and the second elevating/lowering module 650 may be provided between the second transfer module 640 and the second elevating/lowering module 650, and therethrough, the kit gripper 630 according to the embodiment of the present invention may perform horizontal and height movements as well as forward and backward operations.

In addition, the blood sample postprocessing unit 700 may suck a blood sample of the blood collection tube T transferred by the blood sample transfer unit 400 and inject the blood sample into the mounted blood viscosity measurement kit BK.

Specifically, the blood sample postprocessing unit 700 may include an injection device 710, a pipette tip insertion tray 720, a third sliding unit 730, and a third detection unit 740.

First, the injection device 710 may be provided in multiple units and mix and suck a blood sample of the blood collection tube T transferred by the blood sample transfer unit 400 in association with the second transfer module 640 and inject the blood sample into the mounted blood viscosity measurement kit BK.

The injection device 710 may prevent the sedimentation of red blood cells when sucking a blood sample by mixing the blood while sucking and discharging the blood, by using a micropipette technique and through a process of causing the motion motor 230 to shake the blood collection tube insertion plate 210 to the left and right to mix the blood.

In addition, the pipette tip insertion tray 720 may vertically mount a pipette tip PT that is attachable or detachable to or from the end of the injection device 710, and at this time, the pipette tip PT may refer to a disposable pipette tip for safety and accuracy because blood is on something during the test or may refer to a disposable pipette tip of a conductive type to adjust the amount of precise blood suction and discharge required for the test.

In addition, the pipette tip PT may use a precision volume control technology within ±1.0 % according to a change in electrical conductivity, or a precision volume control technology according to a change in pressure by using a disposable tip of a pressure type.

In addition, the third sliding unit 730 may expose the pipette tip insertion tray 720 to the outside by sliding the pipette tip insertion tray 720 to the front of the housing 100.

In addition, a third locking device 731 may be provided on a side surface of the third sliding unit 730 to prevent the third sliding unit 730 from sliding during operation of a device.

In addition, the third detection unit 740 may be provided between the pipette tip insertion tray 720 and the third sliding unit 730, and more specifically, may be provided as a configuration of two-point mounting sensors symmetrically provided on a lower surface of the pipette tip insertion tray 720, but this is only one type of method for effectively detecting horizontal and mounting states of the pipette tip insertion tray 720, and any electronic sensor that may effectively detect the horizontal state and mounting states of the pipette tip insertion tray 720 may be used.

In addition, the automatic blood viscosity measurement device 1 according to the embodiment of the present invention may further include a first waste unit 800 and a second waste unit 900.

First, the first waste unit 800 is provided inside the housing 100 but is provided between the blood sample mounting unit 200 and the blood viscosity measurement unit 500, thereby being able to accommodate a used pipette tip PT.

In addition, the second waste unit 900 is provided inside the housing 100 but is provided between the blood viscosity measuring unit 500 and the kit mounting unit 600, thereby being able to accommodate the blood viscosity measuring kit BK for which measurement is completed.

In addition, the first waste unit 800 and the second waste unit 900 may be exposed to the outside of the housing 100 by sliding forward the housing 100 and be formed in a shape of a sliding drawer like the first, second, and third sliding units 220, 620, and 730, but a locking device (not illustrated) may be provided on a side surface of each of the first and second waste units 800 and 900 to resolve the problem of the first and second waste units 800 and 900 being unexpectedly opened during an operation of the device.

In addition, the automatic blood viscosity measurement device 1 according to the embodiment of the present invention may further include a control unit C, an alarm unit AL, an emergency stop switch unit ES, and a monitoring unit (not illustrated).

First, the control unit C may control operations of the blood sample preprocessing unit 300, the blood sample transfer unit 400, the blood viscosity measurement unit 500, the kit mounting unit 600, and the blood sample postprocessing unit 700.

In addition, the alarm unit AL may detect an error of the automatic blood viscosity measurement device 1 and transmit the error to a user through light or sound.

In addition, the emergency stop switch unit ES may immediately stop an operation of a mechanical device by operating a switch when a user needs an emergency stop of the automatic blood viscosity measurement device 1.

In addition, the monitor unit (not illustrated) may monitor viscosity measurement results measured by the blood viscosity measurement unit 500 through a display device.

Accordingly, the automatic blood viscosity measurement device 1 according to the embodiment of the present invention has an advantage of shortening the blood viscosity test time and enabling continuous testing even when a gripper module or injection device breaks down by applying a configuration of a dual system and has an advantage of preventing sedimentation of red blood cells during the test and enabling safe and quick replacement of the blood collection tube T, the blood viscosity measurement kit BK, and the pipette tip PT.

In conclusion of the description, it is preferable to interpret that the automatic blood viscosity measurement device 1 according to the embodiment of the present invention is not limited to the effects described above and has advantages that may be obviously derived through a combination of the device and configuration of the present invention.

Although the automatic blood viscosity measurement device 1 according to the embodiment of the present invention is described with reference to the attached FIGS. 1 to 19, those skilled in the art to which the present invention pertains will understand that the present invention may be implemented in other specific forms without changing the technical idea or essential features of the present invention. Therefore, the embodiments described above are examples in all aspects and are not restrictive.

### [Reference Signs List]

1: automatic blood viscosity measurement device
100: housing 101: cooling unit
110: opening door 120: frame
200: blood sample mounting unit 210: blood collection tube insertion plate
215: first detection unit 220: first sliding unit
221: first locking device 230: motion motor
300: blood sample preprocessing unit 310: preprocessing gripper
320: first transfer module 330: first lifting/lowering module
340: blood collection tube detection unit 400: blood sample transfer unit
410: holding gripper 420: holding gripper transfer module
430: guide plate 431: guide groove
500: blood viscosity measurement unit 510: measurement unit body
511: vibration-proof pad 520: kit mounting housing
521: kit insertion groove 530: mounting detection sensor
540: heat transfer member 550: kit fixing means
551: first push member 552: second push member
600: kit mounting unit 610: kit insertion tray
615: second detection unit 616: fastening means
620: second sliding unit 621: second locking device
630: kit gripper 640: second transfer module
650: second lifting/lowering module 700: blood sample postprocessing unit
710: injection device 720: pipette tip insertion tray
730: third sliding unit 731: third locking device
740: third detection unit 800: first waste unit
900: second waste unit
T: blood collection tube TC: blood collection tube cover
BK: blood viscosity measurement kit PT: pipette tip
C: control unit AL: alarm unit
ES: emergency stop switch unit

## Claims

1. An automatic blood viscosity measurement device comprising:
a housing including an open door;
a blood sample mounting unit on which multiple blood collection tubes are mounted;
a blood sample preprocessing unit that grips each of the multiple blood collection tubes, scans a gripped blood collection tube, and separates a blood collection tube cover mounted on the gripped blood collection tube;
a blood sample transfer unit that moves the gripped blood collection tube gripped by the blood sample preprocessing unit;
a blood viscosity measurement unit on which a blood viscosity measurement kit is mounted and which includes one or more channels for measuring viscosity of a blood sample injected into the blood viscosity measurement kit;
a kit mounting unit disposed inside the housing for mounting the blood viscosity measurement kit on the blood viscosity measurement unit; and a blood sample postprocessing unit that sucks a blood sample of a blood collection tube transferred by the blood sample transfer unit and injects the blood sample into the mounted blood viscosity measurement kit.

2. The automatic blood viscosity measurement device of claim 1, wherein the blood sample mounting unit comprises:
a blood collection tube insertion plate on which the blood collection tube is vertically mounted;
a first sliding unit that slides the blood collection tube insertion plate to a front of the housing to expose the blood collection tube insertion plate to an outside; and
a first detection unit provided between the blood collection tube insertion plate and the first sliding unit to detect a safe-placement state of the blood collection tube insertion plate.

3. The automatic blood viscosity measurement device of claim 2, wherein the blood sample mounting unit further comprises a motion motor provided on both sides of the blood collection tube insertion plate to prevent sedimentation of red blood cells of the blood sample in the blood collection tube by tilting the blood collection tube insertion plate at a set angle to left and right and repeatedly moving the blood collection tube insertion plate at a set cycle.

4. The automatic blood viscosity measurement device of claim 1, wherein the blood sample preprocessing unit comprises:
a preprocessing gripper provided in multiple units to grip the blood collection tube and the blood collection tube cover to rotate the blood collection tube and the blood collection tube cover;
a first transfer module for adjusting a horizontal position of the preprocessing gripper; and
a first lifting/lowering module causing the preprocessing gripper to be associated with the first transfer module and adjusting a height of the preprocessing gripper.

5. The automatic blood viscosity measurement device of claim 4, wherein the blood sample preprocessing unit further includes a scanning module provided in the first transfer module to scan whether the blood collection tube is mounted on the blood collection tube insertion plate.

6. The automatic blood viscosity measurement device of claim 4, wherein the blood sample transfer unit comprises:
a holding gripper which is provided in multiple units and in which the blood collection tube gripped and transferred by the preprocessing gripper is safely placed and which grips a lower portion of the blood collection tube;
a holding gripper transfer module that transfers the holding gripper; and
a guide plate that guides a reciprocating movement of the holding gripper transferred by the holding gripper transfer module.

7. The automatic blood viscosity measurement device of claim 6, wherein the blood collection tube comprises one or more blood collection tubes, and wherein the blood sample preprocessing unit further includes a blood collection tube detection unit that detects the one or more blood collection tubes gripped by the holding gripper, and
the automatic blood viscosity measurement device automatically measures viscosity of one or more blood samples of the one or more blood collection tubes according to detection information on the one or more blood collection tube.

8. The automatic blood viscosity measurement device of claim 1, wherein the blood viscosity measurement unit comprises:
a measurement unit body having a vibration-proof pad installed at an upper portion of the measurement unit;
a kit mounting housing provided at an upper end of the measurement unit body and having a kit insertion groove in which the blood viscosity measurement kit is mounted;
a mounting detection sensor disposed inside the kit insertion groove and detecting a mounting state of the blood viscosity measurement kit;
a heat transfer member disposed inside the kit mounting housing to apply heat to the blood viscosity measurement kit; and
a kit fixing means disposed inside the kit mounting housing to place at a normal position and fix the blood viscosity measurement kit, and
the kit fixing means comprises:
a first push member provided on an inner surface of the kit insertion groove of the kit mounting housing in a height direction of the inner surface to push the blood viscosity measurement kit to one side; and
a second push member provided on the inner surface of the kit insertion groove of the kit mounting housing in a length direction of the inner surface to push the blood viscosity measurement kit to another side.

9. The automatic blood viscosity measurement device of claim 1, wherein the kit mounting unit comprises:
a kit insertion tray on which the blood viscosity measurement kit is vertically mounted;
a second sliding unit that slides the kit insertion tray to a front of the housing to expose the kit insertion tray to the outside; and
a second detection unit disposed between the kit insertion tray and the second sliding unit to detect a safe-placement state of the kit insertion tray.

10. The automatic blood viscosity measurement device of claim 9, wherein the kit mounting unit further comprises a fastening means disposed between the kit insertion tray and the second sliding unit to fix the kit insertion tray to an upper portion of the kit insertion tray.

11. The automatic blood viscosity measurement device of claim 9, wherein the kit mounting unit is provided in multiple units and comprises:
a kit gripper that grips the blood viscosity measurement kit;
a second transfer module that adjusts a horizontal position of the kit gripper; and
a second lifting/lowering module causing the kit gripper to be associated with the second transfer module and adjusting a height of the kit gripper.

12. The automatic blood viscosity measurement device of claim 11, wherein the blood sample postprocessing unit is provided in multiple units and includes an injection device that mixes and sucks the blood sample of the blood collection tube transferred by the blood sample transfer unit in association with the second transfer module and injects the blood sample into the mounted blood viscosity measurement kit.

13. The automatic blood viscosity measurement device of claim 11, wherein the blood sample postprocessing unit further comprises;
a pipette tip insertion tray on which a pipette tip that is detachable from an end portion of the injection device is mounted; and
a third sliding unit that slides the pipette tip insertion tray to a front of the housing to expose the pipette tip insertion tray to an outside.

14. The automatic blood viscosity measurement device of claim 1, wherein the housing comprises:
a first waste unit provided between the blood sample mounting unit and the blood viscosity measurement unit to accommodate a used pipette tip, and
a second waste unit provided between the blood viscosity measurement unit and the kit mounting unit to accommodate a blood viscosity measurement kit for which measurement is completed, and
the first waste unit and the second waste unit are exposed to an outside of the housing by sliding forward the housing.

15. The automatic blood viscosity measurement device of claim 1, wherein the automatic blood viscosity measurement device further comprises:
a control unit for controlling operations of the blood sample preprocessing unit, the blood sample transfer unit, the blood viscosity measurement unit, the kit mounting unit, and the blood sample postprocessing unit;
an alarm unit for detecting and notifying an error of the automatic blood viscosity measurement device;
an emergency stop switch unit for stopping the automatic blood viscosity measurement device; and
a monitor unit for monitoring a viscosity measurement result of the blood viscosity measurement unit.
